# EUROPEAN PATENT SPECIFICATION

(11) **EP 0 639 382 B1**
(45) Date of publication and mention of the grant of the patent: **31.07.2002**
(21) Application number: 94112724.3
(22) Date of filing: 16.08.1994
(51) Int. Cl.: A61L 26/00

(54) **Cement for medical use, method for producing the cement, and use of the cement**
Medizinischer Zement, Verfahren zu seiner Herstellung sowie dessen Verwendung
Ciment médical, procédé pour fabriquer le ciment, et son utilisation

(30) Priority: 18.08.1993 SE 9302672
(43) Date of publication of application: 22.02.1995
(73) Proprietor: Coripharm GmbH & Co., 64807 Dieburg (DE)
(72) Inventor: Lidgren, Lars Ake Alvar, S-227 31 LUND (SE)
(74) Representative: Wagner, Karl Heinz

(56) References cited:
- EP-A- 0 190 504
- EP-A- 0 242 672
- EP-A- 0 301 759
- EP-A- 0 511 868
- DE-A- 3 730 298
- DE-A- 4 016 135
- FR-A- 2 606 282
- GB-A- 2 156 824
- US-A- 4 668 295

## Description

This invention relates to a method for producing a cement for medical use according to which a liquid component containing a polymerizable substance is combined with a solid component comprising a plastic substance to provide a setting mass to form the cement which is characterized in that prior to combining said liquid and solid components, said plastic substance is mixed with an in vivo non-resorbable, particulate crystalline ceramic material which has been subjected to a sintering process. The invention also relates to a cement for such uses and to the use of set cement as an anchorage for the fixation of prostheses or parts of prostheses or bone and as a substitute material for bone.

The use of anchorage substances in surgical procedures, especially for implants, has led to the development of so-called bone cement. The most common use of bone cement has been to fill the gap between a joint implant and tissue, primarily bone. As the cement sets around an implant immediate fixation will be achieved. This is especially important in the often severly handicapped eiderly group of patients being operated with joint implants. When bone cement is used weight bearing is generally allowed within the first postoperative days. The most important complication hitherto has been infection, joint implants breakage or dislocation. Through strict aseptic and antiseptic measures and methodologic development the most severe problems today are wear of the polymer component and loosening between the metallic and polymer implants and bone leading to an interface failure with bone resorption and loss of implant anchorage. An improvement of material and technique has been going on since joint implant procedure with the bone cement started in the late 1950's through cooperation between researchers, engineers, manufacturers and surgeons.

The most important development in the area of bone cement has been a change of the additives used in bone cement. Various substances with particles between 20-300 micrometers in size have been used. Particulate additive powder has mainly been used in a spherical form. This may be regarded as the accepted technique today. Both resorbable particulate powder such as tri-calcium phosphate and non--resorbable powder such as zirconium oxide have been used. The addition of particulate powder varies between 5-30% with 20-25% regarded as optimum. In contrast to the additives which have a secondary role, for example zirconium oxide or barium sulphate which give a radiopaque bone cement, and tri-calcium phosphate which gives a porous cement, new additives will have to be developed to improve both the biological and mechanical properties of bone cement.

The aim of this invention has been to develop additives which improve the mechanical properties of bone cement and the tissue interface reaction giving a quicker bone or tissue in-/ongrowth to the implant.

A further aim has been to reduce the radioactivity from zirconium oxide but still allow the bone cement to be mixed by modern mixing procedures, which further improves cement quality and reduces monomer exposure in the operating area. Adding crystalline ceramic particulate powder to the polymer methacrylate will resolve some of the problems given above. It is important that a ceramic additive is produced in the classic way in the temperature curve span where the sintering of particulate powder takes place. It excludes dried powder or powder produced through agglumeration. Ceramic powder for bone cement may be produced in two different ways. The first possibility is to produce a synthetic or biological powder with the requested homogeneity followed by sintering in an oven. The sintered beads will then be ground, a procedure that is technically known. After selection of the distribution and size of beads the crystalline ceramic powder is added to the bone cement. The second possibility is to use the above described powder together with water or other solutions in order to get a dough with a microporosity through a special working procedure which dough then will be sent for drying. The mixture will be sintered in the classic way. In the following descripton of the invention the first method is used.

To improve the interface and the biological tissue ingrowth it is according to the invention important that the size of the beads is smaller than those previously used.

At least 50% of the added beads should be less than 50 micrometers with a gaussian curve of distribution and at least 50% of the above additive should be less than 3 micrometers. By using this bead size and distribution the rheological properties of the invented bone cement will be enhanced and the contact area increased at the cement surface. This allows for a rapid tissue ingrowth. It is possible to add further separate crystalline ceramic additives according to the procedure above.

Antibiotics containing bone cement have been used since the beginning of the 70's. The antibiotic granulate is dissolved from the surface of the bone cement. This gives a high antibiotic tissue concentration thereby killing bacteria pre- and postoperatively. The antibiotics may be administered in the bone cement in three different ways. The first way is to add powder to the cement. The second way is to add antibiotics to the seperate crystalline ceramic beads through a pharmaceutical procedure as for instance after soaking in an antibiotic solution. The antibiotics will then be administered into bone cement together with crystalline powder. By this procedure the antibiotics will be at the surface of the additive. This gives the advantage of administering antibiotics with extremely variable acting mechanisms. It is also possible to use a combination of antibiotics both in the bone cement as a powder as well as by integration into the additive ceramic crystalline powder.

The third possibility is to add antibiotics as a solution to the bone cement.

Finally, to avoid absorption of monomer fluid to a considerable extent by the porous particles, the pores can be filled with monomerisable material with a low consistency. After polymerization it might be necessary to grind the material in order to achieve the desired particle size.

The possibility of using cement application in humans today depends on whether or not it could be used in delivery systems, especially in combination with vacuum. This could be achieved by using an appropriate viscosity (rheological properties) of the bone cement. The bone cement defined above will preferably be used together with vacuum and injected into humans through a delivery system with a cement viscosity that allows this procedure.

By using the bone cement according to the invention in a closed system during mixture and delivery no transfer of the bone cement is necessary. One such suitable system is so-called Preepack in which the solid and liquid components are contained separated from each other in different individual chambers within one and the same container, wherein the chambers are connected to each other when the components are to be combined.

By adding heat to the bone cement during the mixing procedure the setting time will be reduced; and by chilling the bone cement the setting time will be increased. By using different temperature intervals it will be possible to individualize the setting time of the above described bone cement according to the invention.

The main area of the above described bone cement is to be used in anchoring joint implants or bone and as a bone substitute.

### Example 1

A synthetically produced hydroxyl apatite powder is mixed with a synthetic zirconium oxide in a proportion of 8:2. The powder is heated to 1250°C with a soft powder shaking procedure. This is followed by a grinding procedure and classification of the powder. As an example 5g of the crystalline ceramic beads thus produced will be added to 35g of bone cement to achieve polymerization and the monomer will be filled separately.

### Example 2

As example 1 with the exception that zirconium oxide is replaced with aluminium oxide.

### Example 3

As in example 1 in which a non-ionic crystalline X-ray contrast aid preferably lohexol is used instead of zirconium oxide.

### Example 4

As in example 1 in which crystalline ceramic calcium phosphate is used instead of zirconium oxide.

### Example 5

As in example 1-4 with the exception that a metallic ceramic powder is used.

### Example 6

As example 1-5 with an antibiotic powder added.

### Example 7

As example 1-6 where the monomer is mixed with an addition of antibiotics.

### Example 8

As in example 1-4 with crystalline ceramic powder which is soaked and dried with antibiotics prior to the mixing.

### Example 9

10g of crystalline particles are soaked with methyl methacrylate containing a polymerization starting system. After curing for 24 hours the material is ground, classified and added to the bone cement powder in order to get 5g of crystalline ceramic in 45g of polymer powder.

## Claims

1. Method for producing a cement for medical use, in which a liquid component comprising a polymerizable substance is combined with a solid component comprising a plastic substance to provide a setting mass which is set to form the cement, **characterized in that** prior to combining said liquid and solid components, said plastic substance is mixed with an in vivo non-resorbable, particulate crystalline ceramic material which has been subjected to a sintering process.

2. Method according to claim 1, **characterized in that** said particulate crystalline ceramic material is chosen among calcium phosphate, zirconium oxide or non--ionic X-ray contrast aid, hydroxyl apatite, alumina, metallic ceramics and combinations thereof.

3. Method according to claim 1 or 2, **characterized in that** said particulate crystalline ceramic material is a mixture of calcium phosphate/alumina or calcium phosphate/zirconium oxide.

4. Method according to any of claims 1-3, **characterized in that** crystalline ceramic calcium phosphate is mixed with a non-ionic X-ray contrast aid, preferably lohexol.

5. Method according to claim 4, **characterized in that** at least 50% of the particulate material has a particle size of less than 20 micrometer.

6. Method according to claim 4, **characterized in that** the particle size distribution of said particulate material follows in general a gaussian curve of distribution.

7. Method according to claim 5 or 6, **characterized in that** at least 50% of the gaussian distributed particles have a particle size of less than 3 micrometer.

8. Method according to claim 1 and 3, **characterized in that** the particulate sintered crystalline ceramic material is mixed with the solution, preferably acrylate, the setting mass is grinded to a particle size wherein at least 50% of the particulate material has a particle size of less than 20 microns, and/or wherein the particle size distribution of said particulate material follows in general a gaussian curve of distribution and/or wherein at least 50% of the gaussian distributed particles have a particle size of less than 3 micrometer.

9. Method according to any of the preceding claims, **characterized in that** an antibiotic or a mixture of antibiotics is contained in either of said solid or liquid components.

10. Method according to any of the preceding claims, **characterized in that** the mixing of said solid and liquid components as well as the application of the setting mass are carried out under vacuum.

11. Method according to any of the preceding claims, **characterized in that** said solid and liquid components are contained or packed in different individual chambers of a common container.

12. Method according to claim 11, **characterized** inthat a communication is provided between said individual chambers so as to bring about the mixing of said solid and liquid components within the common container.

13. Method according to any of the preceding claims, **characterized in that** the setting characteristic of the setting mass is adjusted through supply or withdrawal of heat.

14. Use of a cement as obtained through the method according to any of claims 1-13 for the manufacture of a medicament as an anchoring means to fix prostheses or parts of prostheses.

15. A cement for medical use, comprising a liquid component which comprises a polymerizable substance and a solid component which comprises a plastic substance, wherein said liquid and solid components provide a setting mass which is set to form cement, **characterized in that** said solid component further comprises an in vivo non-resorbable, particulate crystalline ceramic material having been subjected to a sintering process.

16. Cement according to claim 15, **characterized in that** said particulate crystalline ceramic material is selected from the group consisting of calcium phosphate, zirconium oxide, hydroxyl apatite, alumina, metallic ceramics, non-ionic X-ray contrast aid or combinations thereof.

17. Cement according to claim 15 or 16, **characterized in that** said material is an X-ray contrast aid, preferably lohexol.

18. Cement according to any of claims 15-17, **characterized in that** it also contains an antibiotic or a mixture of antibiotics.

19. Cement according to any of claims 15-18, **characterized in that** at least 50% of said material has a particle size of less than 20 micrometer.

20. Cement according to any of claims 15-18, **characterized in that** the particulate crystalline ceramic material has particle sizes distributed in accordance with a gaussian curve of distribution.

21. Cement according to claim 20, **characterized in that** at least 50% of said particulate material has a particle size of less than 3 micrometer.

## Patentansprüche

1. Verfahren zum Herstellen eines Zements für medizinischen Gebrauch, bei dem eine flüssige Komponente, die eine polymerisierbare Substanz enthält, mit einer festen Komponente, die eine Kunststoffsubstanz enthält, kombiniert werden, um eine abbindende Masse zu schaffen, die abbindet, um den Zement zu bilden, **dadurch gekennzeichnet, daß** vor der Kombination der flüssigen und festen Komponenten die Kunststoffsubstanz mit einem kristallinen, nicht resorbierbaren Partikel-Keramikmaterial in vivo gemischt wird, wobei das kristalline Partikel-Keramikmaterial einem Sinterprozeß unterworfen worden ist.

2. Verfahren nach Anspruch 1, **dadurch gekennzeichnet, daß** das kristalline Partikel-Keramikmaterial aus Calciumphosphat, Zirkonoxid oder nichtionischer Röntgenstrahlen-Kontrasthilfe, Hydroxyl-Apatit, Aluminiumoxid, Metallkeramiken und Kombinationen hiervon gewählt ist.

3. Verfahren nach Anspruch 1 oder 2, **dadurch gekennzeichnet, daß** das kristalline Partikel-Keramikmaterial ein Gemisch aus Calciumphosphat/Aluminiumoxid oder Calciumphosphat/Zirkonoxid ist.

4. Verfahren nach einem der Ansprüche 1-3, **dadurch gekennzeichnet, daß** das kristalline Keramik-Calciumphosphat mit einer nicht-ionischen Röntgenstrahlen-Kontrasthilfe, vorzugsweise Lohexol, gemischt ist.

5. Verfahren nach Anspruch 4, **dadurch gekennzeichnet, daß** wenigstens 50 % des Partikelmaterials eine Partikelgröße von weniger als 20 Mikrometer hat.

6. Verfahren nach Anspruch 4, **dadurch gekennzeichnet, daß** die Partikelgrößenverteilung des Partikelmaterials im allgemeinen einer Gaußschen Verteilungskurve folgt.

7. Verfahren nach Anspruch 5 oder 6, **dadurch gekennzeichnet, daß** wenigstens 50 % der gemäß einer Gaußschen Verteilung verteilten Partikel eine Partikelgröße von weniger als 3 Mikrometern haben.

8. Verfahren nach Anspruch 1 und 3, **dadurch gekennzeichnet, daß** das gesinterte kristalline Partikel-Keramikmaterial mit der Lösung, vorzugsweise Acrylat, gemischt wird, wobei die abbindende Masse auf eine Partikelgröße geschliffen wird, wobei wenigstens 50 % des Partikelmaterials eine Partikelgröße von weniger als 20 Mikron besitzen, und/oder wobei die Partikelgrößenverteilung des Partikelmaterials im allgemeinen einer Gaußschen Verteilungskurve folgt und/oder wobei wenigstens 50 % der gemäß einer Gaußschen Verteilung verteilten Partikel eine Partikelgröße von weniger als 3 Mikrometer besitzen.

9. Verfahren nach einem der vorhergehenden Ansprüche, **dadurch gekennzeichnet, daß** ein Antibiotikum oder ein Gemisch aus Antibiotika entweder in der festen oder in der flüssigen Komponente enthalten ist.

10. Verfahren nach einem der vorhergehenden Ansprüche, **dadurch gekennzeichnet, daß** das Mischen der festen und flüssigen Komponenten sowie die Aufbringung der abbindenden Masse unter Vakuum ausgeführt werden.

11. Verfahren nach einem der vorhergehenden Ansprüche, **dadurch gekennzeichnet, daß** die festen und flüssigen Komponenten in verschiedenen einzelnen Kammern eines gemeinsamen Behälters enthalten oder darin verpackt sind.

12. Verfahren nach Anspruch 11, **dadurch gekennzeichnet, daß** zwischen den einzelnen Kammern eine Kommunikation vorgesehen ist, um das Mischen der festen und flüssigen Komponenten in dem gemeinsamen Behälter herbeizuführen.

13. Verfahren nach einem der vorhergehenden Ansprüche, **dadurch gekennzeichnet, daß** die Abbindungseigenschaft der abbindenden Masse durch Zuführen oder Abführen von Wärme eingestellt wird.

14. Verwendung eines Zements, der durch das Verfahren nach einem der Ansprüche 1-13 erhalten wird, für die Herstellung eines Medikaments als Verankerungsmittel zum Befestigen von Prothesen oder Teilen von Prothesen.

15. Zement zur medizinischen Verwendung, der eine flüssige Komponente, die eine polymerisierbare Substanz enthält, und eine feste Komponente, die eine Kunststoffsubstanz enthält, umfaßt, wobei die flüssigen und festen Komponenten eine abbindende Masse schaffen, die. abbindet, um Zement zu bilden, **dadurch gekennzeichnet, daß** die feste Komponente ferner ein kristallines nicht resorbierbares Partikel-Keramikmaterial in vivo umfaßt, das einem Sinterprozeß unterworfen worden ist.

16. Zement nach Anspruch 15, **dadurch gekennzeichnet, daß** das kristalline Partikel-Keramikmaterial aus der Gruppe gewählt ist, die aus Calciumphosphat, Zirkonoxid, Hydroxyl-Apatit, Aluminiumoxid, Metallkeramiken, nichtionischer Röntgenstrahlen-Kontrasthilfe oder Kombinationen hiervon besteht.

17. Zement nach Anspruch 15 der 16, **dadurch gekennzeichnet, daß** das Material eine Röntgenstrahlen-Kontrasthilfe, vorzugsweise Lohexol, ist.

18. Zement nach einem der Ansprüche 15-17, **dadurch gekennzeichnet, daß** er außerdem ein Antibiotikum oder ein gemisch aus Antibiotika enthält.

19. Zement nach einem der Ansprüche 15-18, **dadurch gekennzeichnet, daß** wenigstens 50 % des Materials eine Partikelgröße von weniger als 20 Mikrometer besitzen.

20. Zement nach einem der Ansprüche 15-18, **dadurch gekennzeichnet, daß** das kristalline Partikel-Keramikmaterial Partikelgrößen hat, die gemäß einer Gaußschen Verteilungskurve verteilt sind.

21. Zement nach Anspruch 20, **dadurch gekennzeichnet, daß** wenigstens 50 % des Partikelmaterials eine Partikelgröße von weniger als 3 Mikrometer haben.

## Revendications

1. Procédé pour préparer un ciment à utilisation médicale, dans lequel on combine un composant liquide comprenant une substance polymérisable avec un composant solide comprenant une substance plastique pour obtenir une matière de prise qui prend pour former le ciment, **caractérisé en ce que**, avant de combiner lesdits composants liquide et solide, on mélange ladite substance plastiqué avec une matière céramique cristalline particulaire non résorbable in vivo, qui a été soumise à un procédé de frittage.

2. Procédé selon la revendication 1, **caractérisé en ce que** ladite matière céramique cristalline particulaire est choisie parmi le groupe comprenant le phosphate de calcium, l'oxyde de zirconium pu encore un adjuvant de contraste radiologique non ionique, l'hydroxy-apatite, l'alumine, des céramiques métalliques et leurs combinaisons.

3. Procédé selon la revendication 1 ou 2, **caractérisé en ce que** ladite matière céramique cristalline particulaire est un mélange de phosphate de calcium/alumine ou de phosphate de calcium/oxyde de zirconium.

4. Procédé selon l'une quelconque des revendications 1 3, **caractérisé en ce qu'**on mélange du phosphate de calcium céramique cristallin avec un adjuvant de contraste radiologique non ionique, de préférence du lohexol.

5. Procédé selon la revendication 4, **caractérisé en ce qu'**au moins 50 % de la matière particulaire possède une granulométrie inférieure à 20 micromètres.

6. Procédé selon la revendication 4, **caractérisé en ce que** la distribution granulométrique de ladite matière particulaire suit en général une courbe de distribution gaussienne.

7. Procédé selon la revendication 5 ou 6, **caractérisé en ce qu'**au moins 50 % des particules soumises à une distribution gaussienne possèdent une granulométrie inférieure à 3 micromètres.

8. Procédé selon les revendications 1 et 3, **caractérisé en ce qu'**on mélange la matière céramique cristalline frittée particulaire avec une solution, de préférence de l'acrylate, on broie la matière de prise pour obtenir une granulométrie dans laquelle au moins 50 % de la matière particulaire possède une granulométrie inférieure à 20 microns, et/ou dans laquelle la distribution granulométrique de ladite matière particulaire suit en général une courbe de distribution gaussienne et/ou dans laquelle au moins 50 % des particules soumises à une distribution gaussienne possèdent une granulométrie inférieure à 3 micromètres.

9. Procédé selon l'une quelconque des revendications précédentes, **caractérisé en ce qu'**un antibiotique ou un mélange d'antibiotiques est contenu dans l'un ou l'autre desdits composants solide et liquide.

10. Procédé selon l'une quelconque des revendications précédentes, **caractérisé en ce qu'**on effectue le mélange desdits composants solide et liquide, ainsi que l'application de la masse de prise dans des conditions de vide.

11. Procédé selon l'une quelconque des revendications précédentes, **caractérisé en ce que** lesdits composants solides et liquides sont contenus ou sont conditionnés dans différentes chambres individuelles d'un récipient commun.

12. Procédé selon la revendication 11, **caractérisé en ce qu'**on établit une communication entre lesdites chambres individuelles de façon à obtenir un mélange desdits les composants solide et liquide à l'intérieur du récipient commun.

13. Procédé selon l'une quelconque des revendications précédentes, **caractérisé en ce qu'**on règle les caractéristiques de prise de la matière de prise par apport ou retrait de chaleur.

14. Utilisation d'un ciment tel qu'on l'obtient via le procédé selon l'une quelconque des revendications 1 à 13, pour la préparation d'un médicament à titre de moyen d'ancrage pour fixer des prothèses ou des parties de prothèses.

15. Ciment à utilisation médicale comprenant un composant liquide qui comprend une substance polymérisable et un composant solide qui comprend une substance plastique, dans lequel lesdits composants liquide et solide procurent une matière de prise qui prend pour former un ciment, **caractérisé en ce que** ledit composant solide comprend en outre une matière céramique cristalline particulaire non résorbable in vivo, qui a été soumise à un procédé de frittage.

16. Ciment selon la revendication 15, **caractérisé en ce que** ladite matière céramique cristalline particulaire est choisie parmi le groupe comprenant le phosphate de calcium, l'oxyde de zirconium ou encore un adjuvant de contraste radiologique non ionique, l'hydroxy-apatite, l'alumine, des céramiques métalliques ou leurs combinaisons.

17. Ciment selon la revendication 15 ou 16, **caractérisé en ce que** ladite matière est un adjuvant de contraste radiologique, de préférence du lohexol.

18. Ciment selon l'une quelconque des revendications 15 à 17, **caractérisé en ce qu'**il contient également un antibiotique ou un mélange d'antibiotiques.

19. Ciment selon l'une quelconque des revendications 15 à 18, **caractérisé en ce qu'**au moins 50 % de ladite matière possède une granulométrie inférieure à 20 micromètres.

20. Ciment selon l'une quelconque des revendications 15 à 18, **caractérisé en ce que** la matière céramique cristalline particulaire possède des granulométries distribuées conformément à une courbe de distribution gaussienne.

21. Ciment selon la revendication 20, **caractérisé en ce qu'**au moins 50 % de ladite matière particulaire possèdent une granulométrie inférieure à 3 micromètres.
